# EUROPEAN PATENT SPECIFICATION

(11) **EP 4 539 856 B1**
(45) Date of publication and mention of the grant of the patent: **09.12.2026**
(21) Application number: 23736014.4
(22) Date of filing: 24.06.2023
(51) Int. Cl.: A61K 31/702, A61K 9/00, A61P 1/00, A61P 1/04, A61P 11/00, A61P 31/00

(54) **COMPOSITION OF GALACTOOLIGOSACCHARIDES AND USES THEREOF**
ZUSAMMENSETZUNG AUS GALACTOOLIGOSACCHARIDEN UND VERWENDUNGEN DAVON
COMPOSITION DE GALACTO-OLIGOSACCHARIDES ET UTILISATIONS ASSOCIÉES

(30) Priority: 24.06.2022 GB 202209335
(43) Date of publication of application: 23.04.2025
(73) Proprietor: Clasado Limited, Reading Berkshire RG2 0TD (GB)
(72) Inventor: MARUSZAK, Aleksandra, Reading Berkshire RG2 0TD (GB); HARTHOORN, Leunis Forrinus, Reading Berkshire RG2 0TD (GB); WILLIAMS, Neil, Nottingham Nottinghamshire NG1 4FQ (GB)
(74) Representative: Appleyard Lees IP LLP
(86) International application number: PCT/EP2023/067211
(87) International publication number: WO 2023/247790

(56) References cited:
- WO-A1-2010/122344
- WO-A1-2023/170426
- ROBERTA GRIMALDI ET AL: "Fermentation properties and potential prebiotic activity of Bimuno® galacto-oligosaccharide (65% galacto-oligosaccharide content) on in vitro gut microbiota parameters", BRITISH JOURNAL OF NUTRITION, vol. 116, no. 03, 8 June 2016 (2016-06-08), UK, pages 480 - 486, XP055412358, ISSN: 0007-1145, DOI: 10.1017/S0007114516002269
- ROBERTA GRIMALDI ET AL: "In vitro fermentation of B-GOS: impact on faecal bacterial populations and metabolic activity in autistic and non-autistic children", FEMS MICROBIOLOGY ECOLOGY, vol. 93, no. 2, 16 November 2016 (2016-11-16), pages fiw233, XP055765338, DOI: 10.1093/femsec/fiw233
- RT STAFF: "Prebiotic Found to Help Manage Exercise-induced Bronchoconstriction | RT", 1 September 2016 (2016-09-01), pages 1 - 3, XP093077643, Retrieved from the Internet <URL:https://rtmagazine.com/disorders-diseases/chronic-pulmonary-disorders/asthma/prebiotic-found-help-manage-exercise-induced-bronchoconstriction/> [retrieved on 20230831]
- ANONYMOUS: "Prebiotics could help treat exercise-induced asthma", 7 August 2016 (2016-08-07), pages 1 - 13, XP093077647, Retrieved from the Internet <URL:https://www.medicalnewstoday.com/articles/312132#Prebiotics-and-gut-bacteria> [retrieved on 20230831]

## Description

### Field

The present invention relates to a composition comprising oligosaccharide compounds for use in the prevention, amelioration or treatment of upper respiratory system (URS) and gastro-intestinal (Gl) disorders and/or diseases. The disclosure also relates to a composition comprising oligosaccharide compounds for enhancing performance of athletes. The present invention also relates to the formulation of said composition as dietary supplements and/or medicaments. In particular the present invention relates to an oligosaccharide composition with a relatively high amount of certain beneficial oligosaccharides.

### Background

Human gut microbiota (or microflora) comprises pathogenic, benign and beneficial microbial genera. A predominance of the former may lead to intestinal disorders that can be both acute (e.g. gastroenteritis) and chronic (e.g. inflammatory bowel disease, irritable bowel syndrome and some intestinal cancers). In humans, large intestinal microbiota is acquired at birth. Breast milk-fed infants have a preponderance of bifidobacteria, which easily outcompete other genera of bacteria. This is because human milk components stimulate growth of bifidobacteria. However, many factors can influence the composition and activity of intestinal microbiota over the course of an individual's lifetime, such as environment, drug treatments and diet, in particular the consumption of highly processed foods (see "Role of the gut microbiota in nutrition and health", Valdes, A. M. et al., BMJ, 2018, 361).

Certain components of the gut microbiota have been implicated in the aetiology of gut disease. For example, mycobacteria are associated with Crohn's disease; ulcerative colitis may be triggered by sulphate-reducing bacteria and there may be bacterial involvement in the development of bowel cancer. It would be of benefit if the selective growth of indigenous beneficial gut bacteria could be encouraged by the ingestion of a prebiotic. A prebiotic is a substrate that is selectively utilized by host microorganisms conferring a health benefit. Use of such a prebiotic may have the ongoing beneficial effect of supressing the growth of pathogenic microbiota.

Attempts have been made to influence the balance of the gut microbiota in favour of beneficial microorganisms, such as the bifidobacteria, by adding one or more such microbial strains to an appropriate food vehicle with the intention of conferring a health benefit on the host. Such a live microbial feed supplement is known as a probiotic. However, it is difficult to guarantee the survival of live bacteria in foods after digestion and consequently the actual effectiveness of probiotic may be limited. Therefore prebiotics may be a more promising option for positively affecting gut microbiota in a patient (see Walter J, Maldonado-Gómez MX, Martinez I. "To engraft or not to engraft: an ecological framework for gut microbiome modulation with live microbes", Curr Opin Biotechnol. 2018, 49, 129-139).

As noted above, an alternative approach to dietary manipulation of the gut microbiota is the use of a prebiotic, which is defined as a non-digestible food ingredient that beneficially affects the subject by selectively stimulating the growth and/or activity of one or a limited number of bacteria in the gut, thereby resulting in an improvement in the health of the host.

Upper gastro-intestinal (GI) disorders and/or diseases affect many people around the world, leading to recurrent and discomforting symptoms. For example, upper GI disorders and/or diseases may include heartburn, reflux, gastro-esophageal reflux disease (GERD), gastritis, and dyspepsia.

A number of medicines have been developed to treat upper GI conditions, although none are without drawbacks.

Treatment of upper GI tract conditions may include alkaline agents and gastric acid suppressors and/or the modification of the diet of individuals to certain foods. Antacids, such as aluminium and magnesium hydroxide is commonly used to neutralize gastric acidity, resulting in an increase in stomach and duodenal pH. Antacids are fast acting, but only last for a short duration and do not always prevent heartburn when taken before food or beverages that may provoke symptoms.

H2-receptor antagonists (also known as H2-blockers) are prescription medicaments which can also be used to treat upper GI conditions by inhibiting the action of histamine on the parietal cell, which in turn inhibits acid secretion. Examples of H2-receptor antagonists include ranitidine hydrochloride, cimetidine, nizatidine, rabeprazole, and famotidine. Whilst H2-receptor antagonists are effective, there is a delay between consumption and relief from symptoms and the medicaments costly.

Proton-pump inhibitors ("PPls") are another alternative type of medicine for treating upper GI conditions. PPIs are prescription medicaments which target the amount of gastric acid produced by gastric acid-producing cells. The medications include omeprazole, omeprazole IR, pantoprazole, lansoprazole and esomeprazole. PPIs demonstrate a delay between consumption and relief from symptoms and are costly.

Respiratory disorders and/or diseases typically include coughing, sneezing, headaches, congestion, sore throat, stuffy nose, rhinitis and fever, and the like. Medicaments include liquid elixirs, cough syrups, cold and flu capsules, cold and flu tablets, allergy tablets, effervescent tablets, mouth and nasal sprays, cough drops, and the like.

The most commonly employed medicaments for treating respiratory disorders and/or diseases are ingested or buccally administered to address and/or treat onset of respiratory symptoms. The products typically contain one or more actives dissolved or dispersed in a carrier system for ingestion or buccal delivery into the bloodstream. Typically, respiratory medicaments are the form of cough drops, liquids, or capsules, respiratory products in the form of powders and effervescent tablets. Whilst most medicaments are relatively inexpensive, their effects last for only a limited duration. Some treatments employ combinations of anti-viral and anti-inflammatory compounds. Again the effects of these medicaments are for a limited duration and the inclusion of anti-viral agents greatly increase costs for these types of therapies.

Elite athletes, such as rugby union players, follow physiologically and psychologically demanding training schedules, with frequent competitive matches, limited recovery, and regular international travel. Collectively, these stressors may impair immunity and increase the risk of acute upper respiratory symptoms (URS) (e.g. cough, sneezing, sore throat & nasal congestion) and gastrointestinal symptoms (GIS) (e.g. bloating, belching, flatulence, nausea & diarrhoea) (Peters & Bateman, 1983; Drew et al., 2017; Hellard et al., 2015; Svendsen et al., 2016; Wentz et al., 2018). On average, elite rugby union players experience four episodes of upper respiratory illness and one GI complaint per season (Cunniffe et al., 2009), with the greatest incidences reported during pre-season and winter (Cunniffe et al., 2009; Tiernan et al., 2020; Keaney et al., 2021). Therefore, identifying ways that may reduce these illnesses, or accelerate recovery to allow return to play is imperative for players health and team performance. The profile, genetic material, and functional activity of the gut microbial community (the gut microbiome) have a substantial influence on systemic immune function (Roberfoid et al., 2010). Manipulation of the gut microbiome is possible through dietary intervention, most commonly through consumption of pro- or pre-biotic dietary supplements. Previous studies using single or multi-strain probiotics have reported reductions in URS incidence in active runners (Cox et al., 2007; Gleeson et al., 2011; Strasser et al., 2016). Furthermore, a 2015 Cochrane review reported that probiotics reduced the number and durations of URS episodes in adults and children (Hao et al., 2015). A multi-strain probiotic reduced the incidence and duration of URS by ~2 days in elite rugby union players (Haywood et al., 2013). However, the variety of probiotic strains used across the different studies creates uncertainty as to exactly which is most beneficial for athlete health.

GOS has also previously been shown to reduce the number of URS days and the severity of GIS in a student cohort (Hughes et al., 2011) and those suffering from travellers' diarrhoea (Drakoularakou et al., 2010; Hasle et al., 2017).

One group of compounds that are classified as prebiotics are the galactooligosaccharides, which are galactose-containing oligosaccharides produced from lactose by the transgalactosylase activity of β-galactosidase enzyme. Known galactooligosaccharide prebiotic products contain a mixture of many oligosaccharide compounds in varying proportions, not all of which have the desired beneficial effects on the intestinal microbiota of the consumer. Although such products have shown some beneficial results in improving the health of patients, there remains a need for further improved oligosaccharide compositions to fully realise the potential benefits to consumers of such prebiotics and to provide a long-lasting and inexpensive treatment for URS and GI conditions and/or diseases and/or improving performance of athletes.

WO 2010/122344 A1 discloses the use of a galactooligosaccharide in the prevention or treatment of an inflammatory disorder.

ROBERTA GRIMALDI ET AL: "Fermentation properties and potential prebiotic activity of Bimuno(R) galacto-oligosaccharide (65% galacto-oligosaccharide content) on in vitro gut microbiota parameters", BRITISH JOURNAL OF NUTRITION, vol. 116, no. 03, 8 June 2016 (2016-06-08), pages 480-486 discloses the in vitro potential fermentation properties of a 65 % galacto-oligosaccharide (GOS) content Bimuno^{®} GOS (B-GOS) on gut microbiota composition and their metabolites.

ROBERTA GRIMALDI ET AL: "In vitro fermentation of B-GOS: impact on faecal bacterial populations and metabolic activity in autistic and non-autistic children", FEMS MICROBIOLOGY ECOLOGY, vol. 93, no. 2, 16 November 2016 (2016-11-16), page fiw233 discloses the influence of a prebiotic galactooligosaccharide (B-GOS) on gut microbial ecology and metabolic function using faecal samples from autistic and non-autistic children in an in vitro gut model system.

WO 2023/170426 A1 discloses a prebiotic oligosaccharide composition**.**

### Summary of the Invention

The invention is set out in the appended claims. It is one aim of the present invention, amongst others, to provide a composition comprising oligosaccharide compounds that addresses at least one disadvantage of the prior art, whether identified here or elsewhere, or to provide an alternative to existing compositions. For instance it may be an aim of the present invention to provide a composition comprising oligosaccharide compounds which comprise a higher proportion of specific beneficial oligosaccharides than known oligosaccharide compositions.

According to aspects of the present invention, there is provided a composition as set forth in the appended claims. Other features of the invention will be apparent from the dependent claims, and from the description which follows.

According to a first aspect of the present invention, there is provided a composition comprising oligosaccharide compounds for use in the prevention, amelioration or treatment of one or more of the following upper Gastro-Intestinal (GI) conditions and/or diseases and Upper Respiratory System (URS) conditions and/or diseases: indigestion, food impaction, swallowing difficulties, heartburn, acid reflux, reflux esophagitis, gastroesophogeal reflux disease, gastritis, small intestine bacterial overgrowth, dyspepsia, bacterial respiratory infections, viral respiratory infections, colds, influenza (flu), respiratory allergies, asthma, headache, chilliness, malaise, coughing, sneezing, sore throat, nasal obstruction, and/or upper respiratory tract infection (URTI), wherein the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

A method of preventing, ameliorating or treating URS and GI conditions and/or diseases, which does not form part of the present invention, comprises the administration of a composition comprising oligosaccharide compounds to a subject in need of such prevention, amelioration or treatment, wherein the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

A method of preventing, ameliorating or treating URS and GI conditions and/or diseases in a subject in need of such prevention, amelioration or treatment, which does not form part of the present invention, comprises:
i) determining whether the subject has a URS and GI condition and/or disease and
ii) administering a therapeutically effective amount of a composition comprising oligosaccharide compounds to the subject, wherein oligosaccharide compounds comprise:
   (a) at least 8 wt% Gal-(β1-3)-Gal-(β1-4)-X^{a};
   (b) at least 3 wt% Gal-(β1-3)-Gal-(β1-3)-X^{b}; and
   (c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

Also disclosed is a use of a composition for the manufacture of a medicament for the prevention, amelioration or treatment of URS and GI conditions and/or diseases, wherein composition comprises oligosaccharide compounds, said oligosaccharide compounds comprising:
(a) at least 8 wt% Gal-(β1-3)-Gal-(β1-4)-Xa;
(b) at least 3 wt% Gal-(β1-3)-Gal-(β1-3)-Xb; and
(c) at least 5 wt% Gal-(β1-3)-Gal-(β1-2)-Xc,

based on the total weight of oligosaccharide compounds present in the composition;
wherein Xa, Xb and Xc are each independently selected from monosaccharides.

Also disclosed is a performance enhancing composition for a sports person comprising oligosaccharide compounds, wherein the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

Also disclosed is a composition for use in the modulating the URS and GI environment, wherein the composition comprises oligosaccharide compounds, and the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

As used herein, the terms "treatment", "treating", "treat" and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment" as used herein, covers any treatment of a disease in a mammal, particularly in a human, and includes: (a) preventing the disease from occurring in a subject which can be predisposed to the disease but has not yet been diagnosed as having it; (b) inhibiting the disease, i.e., arresting or slowing its development; and (c) relieving the disease and/or symptoms of the disease, i.e., causing regression of the disease.

The term "subject" used herein includes any human or nonhuman animal. The term "nonhuman animal" includes all mammals, such as nonhuman primates, sheep, dogs, cats, cows, horses.

A "therapeutically effective amount" refers to the amount of compounds, when administered to a subject for treating a disease, is sufficient to effect such treatment for the disease. The "therapeutically effective amount" will vary depending on the compound(s) used, the disease and its severity and the age, weight, etc., of the subject to be treated.

The term, "GI condition and/or disease" refers to a range of conditions and/or diseases associated with the gastro-intestinal tract with identifiable symptoms, including the following upper GI conditions and/or diseases :indigestion, food impaction, swallowing difficulties, heartburn, acid reflux, reflux esophagitis, gastroesophageal reflux disease, gastritis, small intestine bacterial overgrowth and dyspepsia.

The GI condition and/or disease is an upper GI condition and/or disease.

The term, "upper respiratory system condition and/or disease" or "URS system condition and/or disease" refers to a range of conditions and/or diseases associated with the upper respiratory tract with identifiable symptoms, including the following respiratory conditions and/or diseases: bacterial infections, viral infections, colds, influenza (flu), respiratory allergies and asthma.

Preferably the URS condition and/or disease is an upper respiratory tract infection (URTI).

The URS or GI condition and/or disease may be in an individual who is an athlete and/or is undergoing (or about to undergo) physical exercise or training. The composition may be administered prior to, during, or after, undergoing exercise or physical activity. Alternatively, the composition may be administered according to a continuous dosage regime, such as once or twice daily. The therapeutically effective amount may be a standardised dose or a dose determined by individual factors such as age, body mass index (BMI) or the overall weight of the individual.

The term "modulation" or "modulating" refers to the effect of beneficially adjusting the environment of the GI system or URS so as to make it more robust and resilient which in turn reduces medical conditions or severity of such conditions.

The term "performance" refers to the ability of an individual to perform certain physical tasks associated with sports and exercise, including, but not limited to reducing fatigue and improving endurance.

The term "sports person" refers to an individual actively engaged in sports or exercise, including individuals who are professional or amateur.

The present inventors have advantageously found that supplementation using the composition of the invention reduced the duration of URS, the incidence and severity of GIS, and of upper GIS in particular, and enhanced slgA secretory rate in athletes. The composition acts systemically and studies have shown that the composition of the present invention can modulate immune function and reduce illness, which improves an athlete's wellbeing and availability to train and compete.

These types of oligosaccharides having the specified linkages between the galactose units (denoted Gal) and terminal monosaccharides are believed to be particularly beneficial for the maintenance of a healthy human gut microbiota and therefore beneficial to the health of a subject. Known oligosaccharide compositions either do not contain each of these types of oligosaccharides or contain lower amounts of these oligosaccharides. Therefore the compositions of this first aspect provide advantages to the consumer in the establishment and maintenance of human gut microbiota when used as prebiotics. Advantageously and unexpectedly, the compositions of the invention have been found to not only be more actively consumed by the microbiota present in the gut when compared to other oligosaccharides, but during human trials the compositions were also shown to significantly lower URS and GI symptoms.

The weight percentages of the specific oligosaccharide compounds discussed herein are based on the weight of all of the oligosaccharide compounds present in the whole composition. Therefore only the fraction of the composition which is provided by oligosaccharides, either components (a), (b), (c) or any other oligosaccharide present, is taken into account when determining the specified weight percentages. For this determination of oligosaccharide content, disaccharides are included, apart from lactose. The composition of this first aspect may contain other non-oligosaccharide components, including monosaccharides and lactose. These components are not taken into account when determining the specified weight percentages of the oligosaccharide compounds discussed herein. The specified amounts of oligosaccharide compounds in the composition can be referred to as a weight percentage of the oligosaccharide fraction of the composition of this first aspect.

Oligosaccharide compounds (a), (b) and (c) comprise X^{a}, X^{b} and X^{c} respectively, which are each independently selected from saccharides. The X^{a}, X^{b} and X^{c} groups may be considered to be saccharide units. Therefore the groups X^{a}, X^{b} and X^{c} can be considered terminal sugars of the oligosaccharide compounds. The groups X^{a}, X^{b} and X^{c} are suitably independently selected from monosaccharides. Any suitable monosaccharide unit which can form oligosaccharides with the galactose units of compounds (a), (b) and (c) can provide X^{a}, X^{b} and X^{c}. Suitably X^{a}, X^{b} and X^{c} are each independently selected from the following monosaccharides. Suitable monosaccharide units are selected from glucose (Glc), fucose (Fuc), arabinose (Ara), xylose (Xyl), rhamnose (Rha), mannose (Man), galactose (Gal), ribose (Rib), lyxose (Lyx), allose (All), altrose (Alt), gulose (Gul), idose (Ido), talose (Tal), psicose (Psi), fructose (Fru), sorbose (Sor), tagatose (Tag), galactosamine (GaIN), glucosamine (GIcN) and N-Acetylglucosamine (GIcNAc) or a mixture thereof. Therefore each of compounds (a), (b) and (c) may comprise a mixture of oligosaccharide compounds having different respective X groups, for example either Glc or Fuc X groups.

In some embodiments, groups X^{a}, X^{b} and X^{c} are independently selected from the monosaccharides listed above, suitably independently selected from Glc, Fuc, Ara, Xyl, Rha and Man or a mixture thereof.

The saccharide units of the oligosaccharides in the composition of this first aspect may have either the D or the L enantiomeric form. Suitably the Gal saccharide units in components (a), (b) and (c) all have the D enantiomeric form. The components (a), (b) and (c) may therefore be as follows:
(a) D-Gal-(β1-3)-D-Gal-(β1-4)-X^{a};
(b) D-Gal-(β1-3)-D-Gal-(β1-3)-X^{b}; and
(c) D-Gal-(β1-3)-D-Gal-(β1-2)-X^{c}.

Suitably the Gal and the X^{a}, X^{b} and X^{c} saccharide units all have the D enantiomeric form. The components (a), (b) and (c) may therefore be as follows:
(a) D-Gal-(β1-3)-D-Gal-(β1-4)-D-X^{a};
(b) D-Gal-(β1-3)-D-Gal-(β1-3)-D-X^{b}; and
(c) D-Gal-(β1-3)-D-Gal-(β1-2)-D-X^{c}.

In some embodiments, each of X^{a}, X^{b} and X^{c} are Glc. Therefore compounds (a), (b) and (c) may be galactooligosaccharide compounds (GOS) and the composition of this first aspect may be referred to as a galactooligosaccharide composition. Such galactooligosaccharides may be formed by converting lactose to the stated oligosaccharides with a suitable galactosidase enzyme. In such embodiments the components (a), (b) and (c) are suitably as follows:
(a) Gal-(β1-3)-Gal-(β1-4)-Glc;
(b) Gal-(β1-3)-Gal-(β1-3)-Glc; and
(c) Gal-(β1-3)-Gal-(β1-2)-Glc.

Suitably the Gal and the Glc saccharide units all have the D enantiomeric form. The components (a), (b) and (c) may therefore be as follows:
(a) D-Gal-(β1-3)-D-Gal-(β1-4)-D-Glc;
(b) D-Gal-(β1-3)-D-Gal-(β1-3)-D-Glc; and
(c) D-Gal-(β1-3)-D-Gal-(β1-2)-D-Glc.

In some embodiments, each of X^{a}, X^{b} and X^{c} are a mixture of Glc and one or more of other saccharide units described above, for example Fuc, Ara, Xyl, Rha and Man. Therefore each of (a), (b) and (c) may comprise a mixture of oligosaccharide compounds having either Glc or one of the other saccharide units described above as the X group. In such embodiments, components (a), (b) and (c) may be formed by converting a mixture of lactose and an appropriate additional sugar, for example a monosaccharide selected from fucose, arabinose, xylose, rhamnose and mannose, to the oligosaccharides. Suitably each of X^{a}, X^{b} and X^{c} are a mixture of Glc and Fuc.

Other oligosaccharides in the composition comprising oligosaccharide compounds of this first aspect, besides components (a), (b) and (c) discussed above, may also comprise the saccharide unit "X" groups referred to above.

The composition of this first aspect comprises (a) at least 8 wt% Gal-(β1-3)-Gal-(β1-4)-X^{a}. Suitably the composition comprises at least 9 wt% of component (a) or at least 10 wt% of component (a).

Suitably the composition comprises up to 35 wt% of component (a), up to 30 wt% of component (a) or up to 25 wt% of component (a).

Suitably the composition comprises from 8 to 35 wt% of component (a), from 8 to 25 wt% of component (a) or from 10 to 20 wt% of component (a).

The composition of this first aspect comprises (b) at least 3 wt% Gal-(β1-3)-Gal-(β1-3)-X^{b}. Suitably the composition comprises at least 4 wt% of component (b) or at least 5 wt% of component (b).

Suitably the composition comprises up to 25 wt% of component (b), up to 20 wt% of component (b) or up to 15 wt% of component (b).

Suitably the composition comprises from 3 to 25 wt% of component (b), from 4 to 20 wt% of component (b) or from 4 to 10 wt% of component (b).

The composition of this first aspect comprises (c) at least 5 wt% Gal-(β1-3)-Gal-(β1-2)-X^{c}. Suitably the composition comprises at least 6 wt% of component (c).

Suitably the composition comprises up to 25 wt% of component (c), up to 20 wt% of component (c) or up to 15 wt% of component (c).

Suitably the composition comprises from 5 to 25 wt% of component (c), from 5 to 20 wt% of component (c) or from 6 to 10 wt% of component (c).

The above amounts are based on the total weight of oligosaccharide compounds present in the composition.

Suitably in the composition of this first aspect:
component (a) is present in an amount up to 35 wt%;
component (b) is present in an amount up to 25 wt%; and
component (c) is present in an amount up to 25 wt%;
based on the total weight of oligosaccharide compounds present in the composition.

Suitably in the composition of this first aspect:
component (a) is present in an amount from 8 to 25 wt%;
component (b) is present in an amount from 3 to 25 wt%; and
component (c) is present in an amount from 5 to 20 wt%;
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 3 to 25 wt% Gal-(β1-3)-Gal-(β1-3)-Glc; and
(c) from 5 to 20 wt% Gal-(β1-3)-Gal-(β1-2)-Glc,
based on the total weight of oligosaccharide compounds present in the composition.

Suitably the ratio of the wt% of compound (a) to compound (b) is from 1:1 to 3:1, suitably from 1.5:1 to 2.5:1.

Suitably the ratio of the wt% of compound (a) to compound (c) is from 1:1 to 3:1, suitably from 1.5:1 to 2.5:1.

Suitably the ratio of the wt% of compound (b) to compound (c) is from 2:1 to 1:2, suitably from 1.5:1 to 1:1.5.

Suitably, in the composition of this first aspect, the oligosaccharide compounds comprise:
(d) at least 3 wt% Gal-(β1-3)-Gal-(β1-3)-Gal-(β1-4)-X^{d},
based on the total weight of oligosaccharide compounds present in the composition;
   wherein X^{d}, is selected from saccharides.
X^{d} may be selected from the same monosaccharide units described above for X^{a}, X^{b} and X^{c}.

In some embodiments, X^{d} is Glc.

Suitably the Gal saccharide units of component (d) have the D enantiomeric form. Suitably the Gal and the X^{d} saccharide units all have the D enantiomeric form. The components (d) may therefore be as follows: D-Gal-(β1-3)-D-Gal-(β1-3)-D-Gal-(β1-4)-D-X^{d}.

In some embodiments, X^{d} is a mixture of Glc and one or more of other monosaccharide units, for example Fuc, Ara, Xyl, Rha and Man, suitably Fuc.

Suitably the composition comprises at least 4 wt% of component (d) or at least 5 wt% of component (d).

Suitably the composition comprises up to 25 wt% of component (d), up to 20 wt% of component (d) or up to 15 wt% of component (d).

Suitably the composition comprises from 3 to 25 wt% of component (d), from 4 to 20 wt% of component (d) or from 4 to 10 wt% of component (d).

Suitably in the composition of this first aspect:
component (a) is present in an amount from 8 to 25 wt%;
component (b) is present in an amount from 3 to 25 wt%;
component (c) is present in an amount from 5 to 20 wt%; and
component (d) is present in an amount from 3 to 25 wt%;
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 3 to 25 wt% Gal-(β1-3)-Gal-(β1-3)-Glc;
(c) from 5 to 20 wt% Gal-(β1-3)-Gal-(β1-2)-Glc, and
(d) from 3 to 25 wt% Gal-(β1-3)-Gal-(β1-3)-Gal-(β1-4)-Glc,
based on the total weight of oligosaccharide compounds present in the composition.

Suitably, in the composition of this first aspect, the oligosaccharide compounds comprise:
(e) at least 5 wt% Gal-(β1-4)-Gal-(β1-4)-X^{e};
based on the total weight of oligosaccharide compounds present in the composition;
   wherein X^{e} is selected from monosaccharides.
X^{e} may be selected from the same monosaccharide units described above for X^{a}, X^{b} and X^{c}.

Suitably the Gal saccharide units of component (e) have the D enantiomeric form. Suitably the Gal and the X^{e} saccharide units all have the D enantiomeric form. The components (e) may therefore be as follows: D-Gal-(β1-4)-D-Gal-(β1-4)-D-X^{e}.

In some embodiments, X^{e} is Glc.

In some embodiments, X^{e} is a mixture of Glc and one or more of other monosaccharide units, for example Fuc, Ara, Xyl, Rha and Man, suitably Fuc.

Suitably the composition comprises at least 6 wt% of component (e) or at least 7 wt% of component (e).

Suitably the composition comprises up to 25 wt% of component (e), up to 20 wt% of component (e) or up to 15 wt% of component (e).

Suitably the composition comprises from 5 to 25 wt% of component (e), from 5 to 20 wt% of component (e) or from 6 to 10 wt% of component (e).

Suitably in the composition of this first aspect:
component (a) is present in an amount from 8 to 25 wt%;
component (b) is present in an amount from 3 to 25 wt%;
component (c) is present in an amount from 5 to 20 wt%;
component (d) is present in an amount from 3 to 25 wt%; and
component (e) is present in an amount from 5 to 25 wt%;
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 3 to 25 wt% Gal-(β1-3)-Gal-(β1-3)-Glc;
(c) from 5 to 20 wt% Gal-(β1-3)-Gal-(β1-2)-Glc, and
(d) from 3 to 25 wt% Gal-(β1-3)-Gal-(β1-3)-Gal-(β1-4)-Glc, and
(e) from 5 to 25 wt% Gal-(β1-4)-Gal-(β1-4)-Glc;
based on the total weight of oligosaccharide compounds present in the composition.

In the compositions of this first aspect, X^{a}, X^{b}, X^{c}, X^{d} and X^{e} are each independently selected from the monosaccharides described above.

In the compositions of this first aspect, X^{a}, X^{b}, X^{c}, X^{d} and X^{e} may each be independently selected from Glc, Fuc, Ara, Xyl, Rha and Man or a mixture thereof.

In some embodiments, X^{a}, X^{b}, X^{c}, X^{d} and X^{e} are each Glc.

In some embodiments, X^{a}, X^{b}, X^{c}, X^{d} and X^{e} each comprise Fuc. Suitably X^{a}, X^{b}, X^{c}, X^{d} and X^{e} are each a mixture of Glc and Fuc.

The oligosaccharide compounds of the compositions of this first aspect suitably have a relatively high proportion of β1-3 Gal-Gal linkages - mainly due to the presence of the components (a), (b), (c) and optionally (d) in the proportions discussed herein. The inventors have found that such relatively high proportions of β1-3 Gal-Gal linkages may be particularly advantageous for the uses of the composition discussed herein.

Suitably from 35 to 55% of the Gal-Gal linkages in the oligosaccharide compounds are 1-3 linkages, suitably from 40 to 55%, suitably from 40 to 50%.

Suitably from 40 to 60% of the Gal-Gal linkages in the oligosaccharide compounds are 1-4 linkages, suitably from 45 to 55%, suitably from 45 to 52%.

Suitably from 8 to 20% of the Gal-X linkages in the oligosaccharide compounds are 1-3 linkages, suitably from 10 to 18%, suitably from 12 to 16%, suitably wherein X is Glc.

Suitably from 15 to 25% of the Gal-X linkages in the oligosaccharide compounds are 1-4 linkages, suitably from 16 to 24%, suitably from 17 to 22%, suitably wherein X is Glc.

Suitably from 30 to 45% of the Gal-X linkages in the oligosaccharide compounds are 1-2 linkages, suitably from 32 to 43%, suitably from 34 to 41%, suitably wherein X is Glc.

Suitably from 20 to 36% of the Gal-X linkages in the oligosaccharide compounds are 1-6 linkages, suitably from 22 to 34%, suitably from 25 to 32%, suitably wherein X is Glc.

Suitably the Gal-X, linkages referred to above, for example for example Gal-Glu linkages, are β linkages, i.e β-glycosidic bonds.

Suitably the composition comprises at least 25 wt% of trisaccharides, based on the total weight of in the composition. Suitably the composition comprises at least 28 wt% of trisaccharides or at least 30 wt% of trisaccharides.

Suitably the composition comprises up to 70 wt% of trisaccharides, suitably up to 60 wt% trisaccharides or up to 50 wt% trisaccharides.

Suitably the composition comprises from 25 to 70 wt% of trisaccharides, suitably from 30 to 60 wt% trisaccharides or from 30 to 50 wt% trisaccharides.

Suitably the composition comprises at least 10 wt% of tetrasaccharides, based on the total weight of in the composition. Suitably the composition comprises at least 12 wt% of tetrasaccharides or at least 15 wt% of tetrasaccharides. Suitably the composition comprises at least 20 wt% tetrasaccharides or at least 25 wt% tetrasaccharides.

Suitably the composition comprises up to 40 wt% of tetrasaccharides, suitably up to 25 wt% tetrasaccharides or up to 20 wt% tetrasaccharides.

Suitably the composition comprises from 10 to 40 wt% of tetrasaccharides, suitably from 10 to 30 wt% tetrasaccharides or from 12 to 25 wt% tetrasaccharides.

Suitably the composition comprises from 30 to 50 wt% trisaccharides and from 10 to 25 wt% tetrasaccharides, based on the total weight of the composition.

As mentioned above, the determination of disaccharides present in the composition excludes lactose Therefore in some embodiments the composition suitably comprises up to 40 wt% of disaccharides, suitably up to 30 wt% disaccharides or up to 20 wt% disaccharides. Suitably the content of lactose in the composition of this first aspect is minimised. Suitably the composition is substantially free of lactose. Suitably the composition does not contain lactose.

Suitably the composition comprises from 10 to 40 wt% of disaccharides, suitably from 10 to 30 wt% disaccharides or from 10 to 20 wt% disaccharides.

Suitably the composition comprises from 10 to 40 wt% disaccharides, from 30 to 60 wt% trisaccharides and from 10 to 25 wt% tetrasaccharides, based on the total weight of the composition.

The following description relates to the content of disaccharides in the oligosaccharide compounds. Suitably the oligosaccharide compounds comprise up to 40 wt% of disaccharides, suitably up to 30 wt% disaccharides or up to 20 wt% disaccharides.

Suitably the oligosaccharide compounds comprise from 0 to 40 wt% of disaccharides, suitably from 10 to 30 wt% disaccharides or from 10 to 20 wt% disaccharides.

Suitably the oligosaccharide compounds comprise at least 25 wt% of trisaccharides, based on the total weight of oligosaccharide compounds present in the composition. Suitably the oligosaccharide compounds comprise at least 30 wt% of trisaccharides or at least 33 wt% of trisaccharides.

Suitably the oligosaccharide compounds comprise up to 75 wt% of trisaccharides, suitably up to 65 wt% trisaccharides or up to 55 wt% trisaccharides.

Suitably the oligosaccharide compounds comprise from 25 to 75 wt% of trisaccharides, suitably from 30 to 65 wt% trisaccharides or from 34 to 55 wt% trisaccharides.

Suitably the oligosaccharide compounds comprise at least 10 wt% of tetrasaccharides, based on the total weight of oligosaccharide compounds present in the composition. Suitably the oligosaccharide compounds comprise at least 12 wt% of tetrasaccharides or at least 15 wt% of tetrasaccharides.

Suitably the oligosaccharide compounds comprise up to 45 wt% of tetrasaccharides, suitably up to 35 wt% tetrasaccharides or up to 30 wt% tetrasaccharides.

Suitably the oligosaccharide compounds comprise from 10 to 45 wt% of tetrasaccharides, suitably from 10 to 35 wt% tetrasaccharides or from 15 to 30 wt% tetrasaccharides.

Suitably the oligosaccharide compounds comprise from 0 to 40 wt% disaccharides and from 30 to 75 wt% trisaccharides, based on the total weight of oligosaccharide compounds present in the composition.

Suitably the oligosaccharide compounds comprise from 0 to 40 wt% disaccharides, from 30 to 75 wt% trisaccharides and from 10 to 45 wt% tetrasaccharides, based on the total weight of oligosaccharide compounds present in the composition.

The composition of this first aspect suitably comprises at least 50 wt% of oligosaccharide compounds, including components (a), (b), (c) and optionally (d) and (e). Suitably the composition comprises at least 55 wt% of oligosaccharide compounds, suitably at least 60 wt%, based on the total weight of the composition.

Suitably the composition comprises up to 100 wt% oligosaccharide compounds, suitably up to 95 wt%, up to 90 wt% or up to 85 wt% oligosaccharide compounds.

Suitably the composition comprises from 50 to 100 wt% oligosaccharide compounds, suitably from 55 to 95 wt%, or from 60 to 85 wt% oligosaccharide compounds.

In some embodiments, the composition is in the form of a syrup. The syrup suitably comprises at least 50 wt% of oligosaccharide compounds, at least 55 wt% of oligosaccharide compounds, or at least 60 wt%. Suitably the syrup comprises from 50 to 75 wt% of oligosaccharide compounds, suitably from 55 to 70 wt% or from 60 to 70 wt% oligosaccharide compounds.

The syrup may comprise a significant amount of monosaccharides, for example glucose and/or galactose. The syrup may comprise from 15 to 30 wt% monosaccharides, suitably from 20 to 28 wt% monosaccharides or from 21 to 25 wt% monosaccharides, for example glucose and/or galactose.

The syrup suitably comprises from 20 to 30 wt% water, suitably from 22 to 28 wt% water.

The syrup may also contain lactose, for example 4 to 14 wt% lactose.

In some embodiments, the composition is in the form of a powder. The powder suitably comprises at least 60 wt% of oligosaccharide compounds, suitably at least 70 wt% or at least 75 wt% of oligosaccharide compounds. Suitably the powder comprises from 60 to 100 wt% of oligosaccharide compounds, suitably from 70 to 95 wt% or from 75 to 90 wt% oligosaccharide compounds.

The powder suitably comprises a reduced amount of monosaccharides, for example glucose and/or galactose, compared to the syrup discussed above. The powder may comprise from 1 to 10 wt% monosaccharides, suitably from 2 to 8 wt% monosaccharides or from 3 to 7 wt% monosaccharides, suitably approximately 5 wt%, for example of glucose and/or galactose.

The powder suitably comprises from 1 to 10 wt% water, suitably from 3 to 6 wt% water.

The composition of this first aspect may have been purified to remove monosaccharides and optionally disaccharides from the composition.

The composition of this first aspect may have been fractionated to separate the oligosaccharide components of the composition according to their molecular weight, for example to remove disaccharides from the composition or to isolate trisaccharides from the other oligosaccharide components. This may be carried out by any suitable method known in the art, for example high-performance liquid chromatography. The composition produced by such a fractionation may be referred to as an oligosaccharide fraction or a GOS fraction.

In such embodiments, the composition (or oligosaccharide fraction) suitably comprises at least 70 wt% of trisaccharides, tetrasaccharides and higher oligosaccharides, suitably at least 80 wt% or at least 90 wt%. Such higher oligosaccharides have a degree of polymerisation of 5 and above. Suitably the composition comprises at least 95 wt% of trisaccharides, tetrasaccharides and higher oligosaccharides. Suitably the composition consists or consists essentially of trisaccharides, tetrasaccharides and higher oligosaccharides.

In such embodiments, the composition suitably comprises from 40 to 70 wt% of trisaccharides, suitably from 45 to 70 wt% trisaccharides or from 50 to 70 wt% trisaccharides.

Suitably the oligosaccharide compounds comprise from 15 to 50 wt% of tetrasaccharides, suitably from 15 to 40 wt% tetrasaccharides or from 20 to 40 wt% tetrasaccharides.

Suitably the oligosaccharide compounds comprise from 5 to 25 wt% of higher oligosaccharides, suitably from 5 to 20 wt% higher oligosaccharides or from 10 to 20 wt% higher oligosaccharides.

Suitably the composition comprises from 40 to 70 wt% trisaccharides, from 15 to 40 wt% tetrasaccharides and 5 to 25 wt% of higher oligosaccharides, based on the total weight of the composition. Suitably the composition comprises from 50 to 70 wt% trisaccharides, from 20 to 40 wt% tetrasaccharides and from 10 to 20 wt% higher oligosaccharides, based on the total weight of the composition.

In such embodiments the composition (or oligosaccharide fraction) suitably comprises the components (a), (b) and (c) as described above in the following amounts:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},
based on the total weight of oligosaccharide compounds present in the composition;
wherein X^{a}, X^{b} and X^{c} are each independently selected from monosaccharides.

Suitably the composition (or oligosaccharide fraction) comprises the components (a), (b) and (c) as described above in the following amounts:
(a) at least 10 wt% Gal-(β1-3)-Gal-(β1-4)-X^{a};
(b) at least 5 wt% Gal-(β1-3)-Gal-(β1-3)-X^{b}; and
(c) at least 7 wt% Gal-(β1-3)-Gal-(β1-2)-X^{c}.

The composition may comprise components (d) and/or component (e) as described above.

The composition suitably comprises said components in the ratios discussed above.

In some embodiments, the composition of this first aspect is a trisaccharide and tetrasaccharide fractionated product (which may be referred to as a DP3/DP4 fraction).

In such embodiments, the composition (or DP3/DP4 fraction) suitably comprises at least 70 wt% of trisaccharides and tetrasaccharides, suitably at least 80 wt% or at least 90 wt%. Suitably the composition consists or consists essentially of trisaccharides and tetrasaccharides.

In such embodiments, the composition suitably comprises from 50 to 80 wt% of trisaccharides, suitably from 55 to 75 wt% trisaccharides or from 60 to 75 wt% trisaccharides.

Suitably the oligosaccharide compounds comprise from 20 to 50 wt% of tetrasaccharides, suitably from 25 to 45 wt% tetrasaccharides or from 25 to 40 wt% tetrasaccharides.

Suitably the composition comprises from 50 to 80 wt% trisaccharides and from 20 to 50 wt% tetrasaccharides, based on the total weight of the composition. Suitably the composition comprises from 60 to 75 wt% trisaccharides and from 25 to 40 wt% tetrasaccharides, based on the total weight of the composition.

In such embodiments the composition (or oligosaccharide fraction) suitably comprises the components (a), (b) and (c) as described above in the following amounts:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

Suitably the composition (or oligosaccharide fraction) comprises the components (a), (b) and (c) as described above in the following amounts:
(a) at least 10 wt% Gal-(β1-3)-Gal-(β1-4)-X^{a};
(b) at least 5 wt% Gal-(β1-3)-Gal-(β1-3)-X^{b}; and
(c) at least 7 wt% Gal-(β1-3)-Gal-(β1-2)-X^{c}.

The composition may comprise components (d) and/or component (e) as described above.

The composition suitably comprises said components in the ratios discussed above.

In some embodiments, the composition of this first aspect is a trisaccharide fractionated product (which may be referred to as a DP3 fraction). Such a trisaccharide fractionated product may be obtained by the known fractionation methods referred to above. Such a composition suitably comprises at least 70 wt% of trisaccharides, suitably at least 80 wt% or at least 90 wt%. Suitably the composition comprises at least 95 wt% of trisaccharides.

In such embodiments the composition (or oligosaccharide fraction) suitably comprises the components (a), (b) and (c) as described above in the following amounts:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from saccharides.

Suitably the composition (or oligosaccharide fraction) comprises the components (a), (b) and (c) as described above in the following amounts:
(a) at least 10 wt% Gal-(β1-3)-Gal-(β1-4)-X^{a};
(b) at least 5 wt% Gal-(β1-3)-Gal-(β1-3)-X^{b}; and
(c) at least 7 wt% Gal-(β1-3)-Gal-(β1-2)-X^{c}.

The composition may comprise component (e) as described above.

The composition suitably comprises said components in the ratios discussed above.

The composition of this first aspect may be for use as, and incorporated into, a food supplement product for ingestion by a consumer Such a product may be selected from the group consisting of dairy products (for example, liquid milk, dried milk powder such as whole milk powder, skimmed milk powder, fat filled milk powders, whey powders, infant formula, ice cream, yoghurt, cheese, fermented dairy products), beverages, sport drinks, infant foods, cereals, bread, biscuits, confectionary, cakes, food supplements, dietary supplements, animal feeds, poultry feeds or indeed any other food or beverage.

The composition of this first aspect may be incorporated into a synbiotic composition. Such a synbiotic composition is suitably a mixture comprising live microorganisms and substrate(s) selectively utilized by host microorganisms that confers a health benefit on the host, i.e. a probiotic and a prebiotic.

The composition of this first aspect may be, for use as, and in the form of, a pharmaceutical or nutraceutical composition comprising at least one carrier, excipient, or diluent.

Suitable further components of pharmaceutical or nutraceutical compositions and methods of preparing such pharmaceutical or nutraceutical compositions are known in the art.

The composition may be administered in a single dose or in multiple doses. A suitable frequency of administration may be at least once per day, every other day, once per week, once every two, three, or four weeks, once every month, two months, or once every three to six months. The composition may be administered over a period of at least a week, at least a month, at least three to six months, at least one, two, three, four, or five years, or over the course of the disease, or the lifetime of the subject.

It will be apparent to the skilled addressee that the administration of the composition will be optimised during clinical trials.

Compositions of the invention can be formulated into pharmaceutical compositions by combination with appropriate pharmaceutically acceptable carriers, pharmaceutically acceptable diluents, or pharmaceutically acceptable excipients, and can be formulated into preparations in solid, semi-solid, or liquid forms, such as tablets, capsules, powders, granules and solutions.

Pharmaceutically acceptable carriers, excipients, or diluents may include, for example: water, saline, dextrose, maltodextrin, glycerol, ethanol, a salt, e.g., NaCl, MgCl₂, KCI, MgSO₄, etc.; a buffering agent, e.g., a phosphate buffer, a citrate buffer, a Tris buffer, N-(2- Hydroxyethyl)piperazine-N'-(2-ethanesulfonic acid) (HEPES), 2-(N- Morpholino)ethanesulfonic acid (MES), 2-(N-Morpholino)ethanesulfonic acid sodium salt (MES), 3-(N-Morpholino)propanesulfonic acid (MOPS), N-tris[Hydroxymethyl]methyl-3- aminopropanesulfonic acid (TAPS), etc.; a solubilizing agent; a detergent, e.g., a non-ionic detergent such as Tween-20, etc.; glycerol; and the like.

Pharmaceutically acceptable carriers, excipients and diluents are nontoxic to recipients at the dosages and concentrations employed, and can for example include buffers such as phosphate, citrate, and other organic acids; antioxidants including ascorbic acid, glutathione, cysteine, methionine and citric acid; preservatives (such as ethanol, benzyl alcohol, phenol, m-cresol, p-chlor-m-cresol, methyl or propyl parabens, benzalkonium chloride, or combinations thereof); amino acids such as arginine, glycine, ornithine, lysine, histidine, glutamic acid, aspartic acid, isoleucine, leucine, alanine, phenylalanine, tyrosine, tryptophan, methionine, serine, proline and valine, and combinations thereof; monosaccharides, disaccharides and other carbohydrates; low molecular weight (less than about 10 residues) polypeptides; proteins, such as gelatin or serum albumin; chelating agents such as EDTA; sugars such as trehalose, sucrose, lactose, glucose, mannose, maltose, galactose, fructose, sorbose, raffinose, glucosamine, N-methylglucosamine, galactosamine, and neuraminic acid; and/or non-ionic surfactants such as Tween, Brij Pluronics, Triton-X, or polyethylene glycol (PEG).

For oral preparations, the composition of the invention may include appropriate additives to make tablets, powders, granules or capsules, for example, with 30 conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The pharmaceutical composition can be in a liquid form, a lyophilized form or a liquid form reconstituted from a lyophilized form, wherein the lyophilized preparation is to be reconstituted with a sterile solution prior to administration. The standard procedure for reconstituting a lyophilized composition is to add back a volume of pure water (typically equivalent to the volume removed during lyophilization).

A tonicity agent can be included in the formulation to modulate the tonicity of the formulation. Exemplary tonicity agents include sodium chloride, potassium chloride, glycerin and any component from the group of amino acids, sugars as well as combinations thereof. In some embodiments, the aqueous formulation is isotonic, although hypertonic or hypotonic solutions can be suitable. The term "isotonic" denotes a solution having the same tonicity as some other solution with which it is compared, such as a physiological salt solution or serum.

The composition may modulate the abundance of a bacterial genus present in the upper GI tract. In some embodiments, the composition modulates the abundance of a bacterial genus present in one or both of the small intestine or large intestine. In some embodiments, the composition modulates the abundance of a bacterial genus predominant in the small intestine selected from the group of genus *Achromobacter, Agrobacterium, Blautia, Burkholderia, Coprococcus, Cryocola, Enterococcus, Eubacterium, Holdemania, Lactococcus, Mycobacterium, Pseudoramibacter, Ralstonia, Sphingomonas, Streptococcus,* and *Turicibacter.*

A method of preparing a composition comprising oligosaccharide compounds according to the first aspect, comprises the steps of:
(i) providing a source of saccharide compounds;
(ii) treating the source of saccharide compounds with one or more galactosidase enzyme to at least partially convert the source of saccharide compounds to the oligosaccharide compounds.

Suitably the steps of the method are carried out in the order step (i) followed by step (ii).

Suitably the source of saccharide compounds comprises lactose, lactulose or epilactose. Suitably the source of saccharide compounds comprises lactose. The source of saccharide may be lactose, for example a lactose syrup which may be derived from cow's milk. The lactose may be heat treated.

Optionally, no further saccharides, such as monosaccharides or disaccharides are added to the source of saccharide. In such cases, the method produces galactooligosaccharide compounds.

Optionally, the source of saccharide compounds comprises at least one additional saccharide. Suitably the at least one additional saccharide provides the oligosaccharide compounds with an alternative terminal monosaccharide unit, as discussed above. Suitably the at least one additional saccharide is a source of such a monosaccharide unit. The at least one additional saccharide which is a source of such a monosaccharide unit may be a monosaccharide or may be a higher sugar, such as a disaccharide. The at least one additional saccharide may be a source of a monosaccharide selected from glucose (Glc), fucose (Fuc), arabinose (Ara), xylose (Xyl), rhamnose (Rha), mannose (Man), galactose (Gal), ribose (Rib), lyxose (Lyx), allose (All), altrose (Alt), gulose (Gul), idose (Ido), talose (Tal), psicose (Psi), fructose (Fru), sorbose (Sor), tagatose (Tag), galactosamine (GaIN), glucosamine (GIcN) and N-Acetylglucosamine (GlcNAc) or a mixture thereof. The at least one additional saccharide may be one or more of the monosaccharides listed above.

In this case the method produces oligosaccharides having one or more of the above monosaccharides as the terminal sugar unit.

The at least one additional saccharide may be selected from Fucose, Arabinose, Xylose, Rhamnose, Mannose or mixtures thereof. The source of saccharides may comprise lactose and one or more sources of said monosaccharides. In this case the method produces oligosaccharides having as a terminal sugar unit selected from Glc, Fuc, Ara, Xyl, Rha and Man, or mixtures thereof.

Step (ii) of the method involves treating the source of saccharide compounds with at least one galactosidase enzyme. The galactosidase enzyme may be an alpha- or beta-galactosidase enzyme, depending on whether alpha or beta linkages between the saccharide units of the oligosaccharide compounds is required. Suitably the enzyme exhibits galactosyltransferase (transgalactosidic) activity and forms alpha- or beta- linkages between sugar units in the source of saccharide compounds. This results in the synthesis of oligosaccharide compounds with two or more galactose units derived from lactose. Suitably step (ii) is carried out until conversion of the source of saccharides to the oligosaccharide compounds is complete.

Step (ii) may involve treating the source of saccharide compounds with one or more additional enzymes which are not galactosidase enzymes.

Suitably the method comprises a step (iii) of separating the galactosidase enzyme from the composition comprising oligosaccharide compounds. Step (iii) may involve removing the enzyme by filtration, for example by nanofiltration.

The composition comprising oligosaccharide compounds produced in step (iii) may be heat treated.

Optionally, the composition is evaporated to reduce the water content to provide the final composition comprising oligosaccharide compounds as a syrup, as discussed above in relation to the first aspect.

Optionally, glucose is removed from the composition produced by step (iii) before evaporation. This suitably lowers the glucose content of the composition from 20-30 wt% to below 10 wt%, suitably approximately 5 wt% or lower. The water content of the composition is then reduced by evaporation and the product dried to provide the final composition comprising oligosaccharide compounds as a powder, as discussed above in relation to the first aspect.

It will also be appreciated that a person skilled in the relevant art could produce a composition comprising oligosaccharide compounds according to the first aspect, i.e. containing the specified amounts of particular oligosaccharide compounds, by combining said oligosaccharides obtained and isolated from different sources in the required amounts.

The invention is described below, by way of example only, with reference to the accompanying figures in which:
Figure 1 is a graph illustrating the results of the butyrate analysis across three donors after being administered the oligosaccharide of the present invention alongside a comparative oligosaccharide;
Figure 2 is a graph illustrating the weekly GIS scores reported during 24-week study (the composition comprising oligosaccharide oligosaccharide compounds of the present invention (herein after also referred to as B-GOS)) n = 16, Placebo n = 17), data presented as mean ± SD. AUC analysis revealed difference between-groups differences (P < 0.05);
Figure 3 is a graph illustrating the saliva IgA concentration before and after 12 and 24 weeks of the study (B-GOS n = 16, Placebo n = 17), data presented as median (IQR). Asterisk (*) denotes significant difference between groups (P < 0.05); and
Figure 4 is a graph illustrating the saliva IgA secretion rate before and after 12 and 24 weeks of the study. (B-GOS n = 16, Placebo n = 17), data presented as mean ± SD. Asterisk (*) denotes significant difference between groups (P < 0.05).

### Examples

### Example 1 - oligosaccharide syrup

Example 1 is a composition comprising oligosaccharide compounds, according to the present invention, in the form of a syrup. Such compositions may be obtained by the following procedure, which does not form part of the present invention. Lactose was rehydrated with potable water to give a working solution of between 35-65 wt% solids. The lactose solution was heat treated then cooled to 40-65°C. The pH of the solution was adjusted to pH 5.5-7.5. A beta-galactosidase enzyme was then added to the solution in a closed vessel and subsequently allowed to react with the lactose to catalyse the transfer of galactose molecules to produce oligosaccharide compounds. The progress of the reaction was monitored by measuring the generation of glucose. The reaction was allowed to proceed for between 8 and 26 hours. The reaction was then terminated by high heat treatment. The reaction mixture was cooled and filtered by carbon filtration to remove the enzyme. The mixture was then dried by evaporation to reduce the water content to approximately 22-28 wt% to provide the product as a syrup.

### Example 2 - oligosaccharide powder

Example 2 is a composition comprising oligosaccharide compounds, according to the present invention, in the form of a powder. Such compositions may be obtained by a modification of the procedure described above, which does not form part of the present invention. After removal of the enzyme, the mixture was further filtered to remove a significant portion of the glucose and other monosaccharides, reducing the monosaccharide content from around 23 wt% to around 5 wt%. The water content of the composition was then reduced by evaporation and the product dried to provide a powder having a water content to approximately 3-6 wt%.

### Example 3 - fractionation

Isolation of the different fractions (DP2, DP3, DP4 or DP5) from a sample of Example 2 was performed using a 5 × 70 cm BioGel P2 column, using water as eluent. The column was operated with a flow speed of 40 to 100 mL/h at room temperature (21 °C). Depending on the run, 0.5, 0.75, 1.0, 1.5 or 2.0 mL of a 0.5 g/mL solution of Example 2 in ultrapure water was loaded. The column was loaded in total 15 times with this solution to obtain enough material of the low abundant fractions, i.e. DP5. After a void volume of around 725 mL, 5 mL fractions were collected. Analysis using thin layer chromatography (TLC) and HPAEC-PAD were done to ensure molecules with the same DP are pooled.

Pooled fractions were frozen and lyophilized. The dry material of all runs was combined and redissolved. After a second cycle of freezing and lyophilization, the dry material was stored at 4 °C for further analyses or experimentation.

### Comparative Example 1

A commercially available composition comprising oligosaccharide compounds was obtained in powder form.

### Oligosaccharide analysis

Samples of Example 2 of the present invention and Comparative Example 1 were analysed to determine their oligosaccharide content by the following procedure.

### Materials and Methods

Samples of dry powder of each of Example 2 and Comparative Example 1 were dissolved in water to provide solutions having a concentration of 40 g/l for analysis.

### Gel permeation chromatography

An HPLC apparatus equipped with a Rezex RSO and a RI detector and in-line desalting (for removal of salts and charged material like proteins) was used for the aqueous GPC separation of the components of the samples. The separation was performed at elevated temperature (80°C). The separation range of the Rezex RSO column is from DP1 (monosaccharide) up to about DP10. All samples were analysed undiluted (at 40 g/L). Before analysis, all sample solutions were treated at 100°C for ten minutes in order to remove any microbiological or enzymatic activity.

### GOS fingerprinting

HPAEC-PAD (high performance anion exchange chromatography) equipped with a PA-1 column was used for separation of mono- and oligosaccharides of the different samples. Efforts were made to achieve a separation quality described in van Leeuwen et al., Carbohydrate Research 2016, 425, 48-58. A commercial maltooligosaccharide mixture and Comparative Example 1 were also injected for comparison of chromatograms with those reported by Van Leeuwen et al. Based on this, peak annotations were made for a number of peaks. The samples as used for GPC were 100-fold diluted with DMSO before injection.

### Results

### Gel permeation chromatography (GPC-RI)

Table 1 shows DP (degree of polymerisation) composition results for the samples using Rezex-RSO system, based on RI calibration with glucose (values expressed as g/L in the samples). All material eluting in the >DP5 window was combined.

**Table 1**

| | Comp. Ex. 1 | | Example 2 | |
|---|---|---|---|---|
| Fraction | Conc. (g/l) | wt% | Conc. (g/l) | wt% |
| DP>5 | 1.1 | 2.8 | 1.6 | 4.2 |
| DP=5 | 2.2 | 5.6 | 2.6 | 6.9 |
| DP=4 | 5.1 | 13.1 | 6.1 | 16.1 |
| DP=3 | 10.7 | 27.4 | 12.6 | 33.2 |
| DP=2 | 17.8 | 45.6 | 13.1 | 34.6 |
| glucose | 2.1 | 5.4 | 1.7 | 4.5 |
| galactose | 0.1 | 0.3 | 0.2 | 0.5 |
| Total | 39.0 | 100.0 | 37.9 | 100.0 |
| Total DP 2-5 | 35.7 | | 34.4 | |

The concentration information can be used to calculate the relative weight percentages of the different DP fractions of oligosaccharides contained in the samples, as shown in Table 1, wherein DP=2 refers to disaccharides, DP=3 refers to trisaccharides etc.

### GOS fingerprinting

To identify the individual galactooligosaccharides in the sample (GOS fingerprinting) a gradient was developed giving comparable separation to that reported in van Leeuwen et al., Carbohydrate Research 2016, 425, 48-58. Peak annotations were made in the chromatograms of all GOS samples based on peak annotations made in van Leeuwen et al. for the oligosaccharide compounds. Retention windows of about 15 seconds were applied for peak annotation.

Table 2 shows information on HPAEC-PAD peak areas of all annotated peaks together with information on incubation conditions, sample concentration, dilution and injection volume as shown.

**Table 2**

| | Comp. Ex. 1 | | Example 2 | |
|---|---|---|---|---|
| Compound ID | Peak areas | wt% | Peak areas | wt% |
| Unknown 1 | 1.6 | 0.6 | 0.5 | 0.2 |
| Unknown 2 | 1.5 | 0.5 | 1.9 | 0.7 |
| Galactose | 0.5 | 0.2 | 1.3 | 0.5 |
| Glucose | 19.5 | 7.0 | 17.5 | 6.7 |
| Unknown DP1 | 0.6 | 0.2 | 1.8 | 0.7 |
| Unknown 3 | 1.5 | 0.5 | 1.2 | 0.5 |
| 3 | 0.2 | 0.1 | 0.9 | 0.3 |
| Allolactose | 16.2 | 5.8 | 24 | 9.1 |
| Lactose | 48.4 | 17.5 | 30.9 | 11.8 |
| 6 | 7.2 | 2.6 | 2.1 | 0.8 |
| Unknown DP3 | 3.5 | 1.3 | 0.8 | 0.3 |
| 8a | 48.5 | 17.5 | 38.5 | 14.7 |
| 8b | 18.1 | 6.5 | 14.6 | 5.6 |
| 9 | 7.9 | 2.9 | 8.1 | 3.1 |
| 10 | 16 | 5.8 | 13.1 | 5.0 |
| 11 | 27.6 | 10.0 | 20.4 | 7.8 |
| 12 | 1.4 | 0.5 | 28.9 | 11.0 |
| 13 | 20.4 | 7.4 | 4.3 | 1.6 |
| 17 | 11.8 | 4.3 | 2.7 | 1.0 |
| 18 | 7.1 | 2.6 | 0.9 | 0.3 |
| 22 | 6.4 | 2.3 | 3.9 | 1.5 |
| 23 | 1.9 | 0.7 | 4.6 | 1.8 |
| 24 | 1.1 | 0.4 | 2 | 0.8 |
| 29 | 4.7 | 1.7 | 12.8 | 4.9 |
| 30 | 2.6 | 0.9 | 12.6 | 4.8 |
| 31 | 0.8 | 0.3 | 12.3 | 4.7 |
| sum | 277.1 | 100.0 | 262.5 | 100.0 |

The peak areas were assumed to approximately correspond to the amount of each oligosaccharide compound present in the composition. Where a particular oligosaccharide compound was not identified then "unknown" and a number is entered in the table. The identified compounds are either identified by name or by a number which corresponds to the number assigned to particular galactooligosaccharides in van Leeuwen et al., Carbohydrate Research 2016, 425, 48-58. A list of these galactooligosaccharides and their corresponding numbers is provided below.

The oligosaccharide components (a)-(e) discussed above, wherein each X group is Glu, correspond to the following numbered entries in Table 2 above:
(a) D-Gal-(β1-3)-D-Gal-(β1-4)-D-Glu - **12**
(b) D-Gal-(β1-3)-D-Gal-(β1-3)-D-Glu - **29**
(c) D-Gal-(β1-3)-D-Gal-(β1-2)-D-Glu - **30**
(d) D-Gal-(β1-3)-D-Gal-(β1-3)-D-Gal-(β1-4)-D-Glu - **31**
(e) D-Gal-(β1-4)-D-Gal-(β1-4)-D-Glu - **11.**

### Prebiotic effects - butyrate production

Butyrate is produced by gut microbiota which convert acetate and/or lactate (along with other substrates) to butyrate. As butyrate is a secondary metabolite, it is often produced during late stages of the incubation. These experiments were aimed at assessing the difference in butyrate production of the compositions of the present invention alongside a comparative GOS composition so as to see if they were more effective as a prebiotic. The experiments used the following procedure.

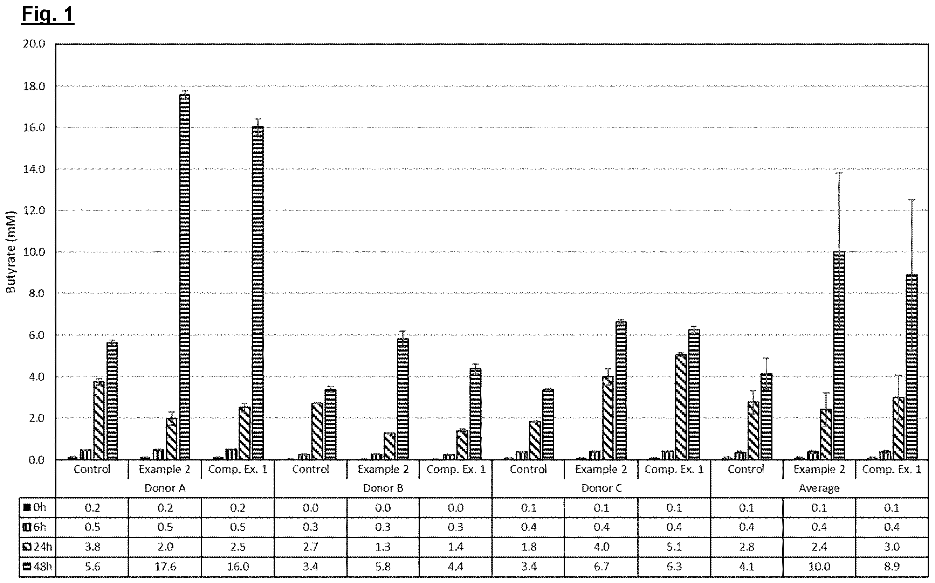

The compositions of Example 2 of the present invention, Comparative Example 1 and a control blank sample were subjected to dialysis using a 0.5 kDa membrane to provide 5 g/l samples which were then mixed with faecal matter obtained from three healthy human adult subjects (donors A, B and C). The mixtures were shaken under anaerobic conditions and monitored over a 48 hour period for colonic fermentation products including butyrate (with 6, 24 and 48 h collection points). The distribution of oligosaccharides in the mixtures was also monitored over this time period using the method described above in relation to Table 2. The results show that Example 2 was well fermented by all donors, mainly during the time period 0-24 hours and that butyrate production increased compared to the Comparative Example 1 and the control at the 6 and 48 hour time points. The results of the butyrate analysis are shown in Figure 1. The results of the oligosaccharides analysis for the samples at the different time points are shown in Table 3. These results show that the oligosaccharides in the samples were actively consumed by the microbiota present in the faecal samples during the experiments.

To assess whether treatment effects on gut microbial activity were statistically significant, three two-sided T-tests were performed between Example 2 and the control, Comparative Example 1 and the control and Example 2 and Comparative Example 1 to obtain p-values. The Benjamini-Hochberg false discovery rate (FDR) was also used in this analysis. Differences between treatment effects were considered significant when the obtained p-value was smaller than a reference value. Table 4 below shows the differences in the averaged butyrate production for the compared samples over 48 hours and the asterisk denotes whether the difference was considered significant according to the analysis described above. These results show that the increase in butyrate production provided by Example 2 during the time period 0-48 hours was statistically significant compared to the control and Comparative Example 1.

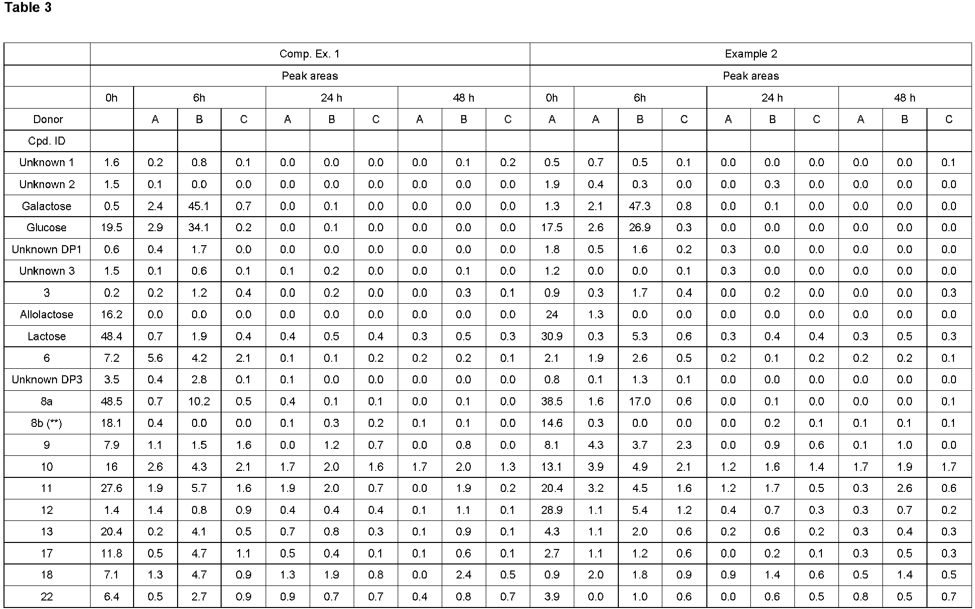

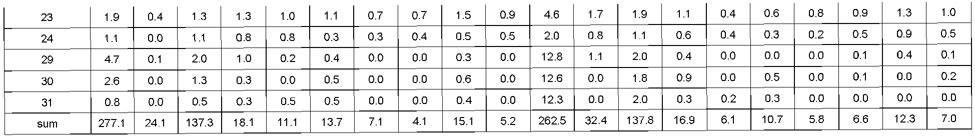

**Table 4**

| D0-48h / Production | Example 2 - Control | Comp. Ex. 1 - Control | Example 2 - Comp. Ex. 1 |
|---|---|---|---|
| Butyrate (mM) | 5.88* | 4.75* | 1.13* |

In summary, the present invention provides a composition comprising oligosaccharide compounds, for example galactooligosaccharide compounds, which includes: (a) at least 8 wt% Gal-(β1-3)-Gal-(β1-4)-X^{a}; (b) at least 3 wt% Gal-(β1-3)-Gal-(β1-3)-X^{b}; and (c) at least 5 wt% Gal-(β1-3)-Gal-(β1-2)-X^{c}, based on the total weight of oligosaccharide compounds present in the composition; wherein X^{a}, X^{b} and X^{c} are each independently selected from monosaccharides. These compositions contain relatively high amounts of the oligosaccharide compounds (a), (b) and (c) and a relatively high amount of β1-3 Gal-Gal linkages, compared to known oligosaccharide compositions. These particular features of the composition are believed to provide benefits to the gut health of a consumer, for example due to these compositions providing an increased production of butyrate in the gut of a consumer compared to known compositions.

### Example 4 - The effects of 24-week prebiotic intervention on self-reported upper respiratory, gastrointestinal symptoms and markers of immunity in elite rugby union players

The study assessed the effects of a 24-week B-GOS supplementation on the severity, duration and incidence of URS and GIS, salivary immunoglobulin A (slgA), and concentrations of C-reactive protein (CRP) and TNF-α in elite rugby union players during a competitive season.

The study was a randomised, double-blind, placebo-controlled trial over the course of 24-weeks during a regular rugby union season in the Gallagher English Premiership. Forty-one healthy, elite rugby union players (mean age 23.5 ± 4.7years; body mass 103.8 ± 13.2kg; 186.5 ± 7.8cm) from a single club volunteered to participate in the present study. All individuals were non-smokers, had no history of gastrointestinal illness and were not regularly consuming foods enriched with probiotics, prebiotics, or vitamins. Participants were matched into pairs based on body mass and playing position before randomly allocated an intervention (see Table 5 below). All data was collected during English autumn and winter months (temperature range -4 to +25°C).

During the study, a typical week for participants included four to five days training (lasting ~5 hours a day), one competitive match and at least one day of rest. Training included resistance, skills, fitness, tactics, and match play exercises. Players not named in the competitive matchday squad would train for an extra day. Participants did not follow individualised diet plans but were provided meals onsite during training days. A cooked breakfast was provided before the first training session with a snack following the morning session. A main meal would then succeed the afternoon session. A similar meal would be provided before a competitive match with a recovery drink post-match. Similarly, no meal plan was provided when away from the training ground. However, individuals were instructed to avoid any foods enriched with probiotics, prebiotics, and vitamins. Before each data collection visit, individuals were asked to arrive following an overnight fast, and to have avoided using mouthwash.

### Supplementation

Players were randomised to consume either 2.9g/day of the oligosaccharide composition of the present invention (in powder form) or 2.9g/day placebo (maltodextrin) . Both supplements were identical in taste and colour, and the supplement was blinded. Researchers, club staff, and participants remained blinded until all statistical analysis was complete. Participants were provided with supplement pre-mixed in water at the training ground and consumed under observation by a member of club staff. On rest days, players were instructed to mix the sachet into water and consume at breakfast. Participants returned used and unused sachets to assess supplement adherence.

### Daily upper respiratory symptoms

To establish the presence of URS, participants completed the Jackson questionnaire daily (Jackson et al., 1958). The presence of 8 symptoms (headache, chilliness, sneezing, sore throat, malaise, cough, nasal discharge, nasal obstruction) were rated on a scale of 0-3 (0-none, 1-mild, 2-moderate, 3-severe). Total symptom scores for each day were summed to give a total Jackson symptom score. An episode of URS was defined using the Jackson criteria as applied by Martineau, Hanfia and Witt (2015), with an episode defined by any period lasting ≥ 3 days with a Jackson score ≥ 14 and the presence of nasal discharge or a symptom score < 14 with a subjective impression of having a cold for at least 3 days. If URS symptoms returned within one week it was regarded as the same episode.

### Weekly gastrointestinal symptoms

To assess gastrointestinal symptoms (GIS), participants were required to complete a weekly gastrointestinal symptom tool (Gaskell et al. 2019). Participants were educated and advised to rate each symptom using the 10-point visual analogue scale, with 1-4 indicative of mild GIS (i.e. sensation of GIS, but not substantial enough to interfere with exercise), 5-9 indicative of severe GIS (i.e. GIS substantial enough to interfere with exercise), and 10 indicating extremely severe GIS (i.e. causing reductions in exercise workload or cessation from exercise). If no specific GIS was reported, this would be rated as zero. GIS including regurgitation, projectile vomiting and defecation were rated as either 0 or 10 only as the presence of these would result in cessation of exercise. Participants were asked to rate each symptom in reference to the previous 7-days. All symptom scores were summed to give a weekly total and incidence. Furthermore, the maximum weekly score possible for everyone was 190 (Gaskell et al., 2019).

### Collection and analysis of slgA

At week 0, 12 and 24 all participants provided a saliva sample to determine slgA. Participants rinsed their mouth using plain water and remained seated for 10-mintes before providing each sample. An unstimulated saliva sample was produced using passive method, where participants were instructed to pool as much saliva as possible in their mouth for 2-mins, then tilt their head forward and release saliva slowly into the collection tube for a further 2-mins. Samples were immediately frozen at -20°C and then at -80°C within 48 hours until analysis. Upon analysis samples were fully thawed at room temperature and slgA concentration was determined by enzyme-linked immunosorbent assay (ELISA) (Salimetrics, Philadelphia, PA). The slgA, intra- and inter-assay variation was 3.2% and 7.3% and the minimum detectable level of the assay was 2.5 µg/ml which all samples exceeded. slgA flow rate was calculated by multiplying the concentration of slgA (µg/ml) by the flow rate (ml/min), resulting in a concentration measure per unit of time (µg/min) as per manufacturer's instructions.

### Collection and analysis of blood biomarkers of systemic inflammation

All participants provided a blood sample at week 0, 12 and 24. Samples were drawn from the antecubital vein in two 10ml vacutainers one heparin and one EDTA coagulant (BD Vacutainer^{®}). Plasma was used to determine tumor necrosis factor-alpha (TNF-α) and CRP concentrations at each time point. Samples were centrifuged, immediately frozen at -20°C and then at -80°C within 48 hours until analysis. TNF-α was assessed using high sensitivity ELISA and CRP was assessed using a regular ELISA protocol (R&D systems). For TNF-α, the intra- and inter-assay variation was 7.5% and 5.8% and the minimum detectable level of the assay was 0.022 pg/ml which all samples exceeded. For CRP, the intra- and inter-assay variation was 4.4% and 7.8% and the minimum detectable level of the assay was 0.022 pg/ml which all samples exceeded.

### Statistical Analysis

Statistical analyses were performed using the statistical package for social sciences (IBM SPSS version 26, Illinois, United States). All data were checked for normal distribution using the Shapiro-Wilk test. The area under the curve (AUC) of daily URS and weekly GIS over the 24-weeks were compared using a Mann-Whitney U test to assess differences in symptom severity. Between group differences in URS incidence were assessed using a Mann-Whitney U test. Differences in URS episode duration and GIS free weeks were assessed using an independent t-test. CRP, TNF-α and slgA secretion rate were evaluated using an analysis of covariance (ANCOVA), adjusting for baseline values. Data evaluated using parametric tests are presented as mean ± standard deviation. Data presented using non-parametric tests are presented as median (range). Statistical significance was set at P < 0.05.

### Player Characteristics

The final sample consisted of 33 participants (n = 17 Placebo, 16 B-GOS) who successfully completed the full 24-week supplementation period. Adherence to the supplement both at training and home was good (B-GOS: 80.4 ± 13.9 %, Placebo: 78.3 ± 14 %, P = 0.73). Body mass, height, age, average weekly workload, and competitive minutes played did not differ between groups (P > 0.05) (See Table 5 showing the characteristics of study participants).

**Table 5**

| **Factor** | **B-GOS** | **Placebo** | **p-value** |
|---|---|---|---|
| **Randomisation N (%)** | 16 (48%) | 17 (52%) | |
| **Age (Years), mean (SD)** | 22.4 (3.3) | 24.5 (5.2) | 0.16 |
| **Height (cm), mean (SD)** | 186.9 (9.4) | 186.6 (7.3) | 0.91 |
| **Body Mass (kg), mean (SD)** | 103.4 (14.0) | 105.2 (13.2) | 0.71 |
| **BMI, mean (SD)** | 29.5 (2.4) | 30.2 (3.1) | 0.49 |
| **Unit - Back** | 8 (50%) | 7 (41%) | 0.61 |
| **Unit - Forward** | 8 (50%) | 10 (59%) | 0.61 |
| **Weekly Workload (AU), mean (SD)** | 672.4 (129.6) | 675.9 (140.4) | 0.93 |
| **Competitive minutes played, mean (SD)** | 657.4 (279.5) | 597.9 (261.3) | 0.57 |

### Upper respiratory Symptoms (URS)

A Mann-Whitney U test revealed no differences in the URS incidence rate between B-GOS (1.0 ± 1.4) and Placebo (1.0 ± 1.0) (P = 0.64) (see Table 6 showing an overview of self-reported URS data). There was no difference in AUC of the daily symptoms scores between the two groups (P = 0.77). The duration of individual URS episodes was shorter in the oligosaccharide group (7.4 ± 2.8 days) compared to the placebo group (9.8 ± 4.1 days) (P = 0.04).

**Table 6**

| | **B-GOS** | | | | **Placebo** | | | | |
|---|---|---|---|---|---|---|---|---|---|
| | **Mean** | **SD** | **Median** | **Range** | **Mean** | **SD** | **Median** | **Range** | **P Value** |
| **Episode Duration (days)** | 7.42 | 2.83 | 7.00 | (4-15) | 9.82 | 4.05 | 10.00 | (4-17) | 0.045 |
| **Number of Episode Days** | 9.88 | 9.74 | 7.00 | (0-34) | 9.71 | 10.1 | 7.00 | (0-30) | 1.000 |
| **Incidence** | 1.00 | 1.44 | 1.00 | (0-5) | 1.00 | 1.00 | 1.00 | (0-3) | 0.641 |
| **Severity** | 43.47 | 27.41 | 35.80 | (14-118) | 59.97 | 34.17 | 50 | (15-138) | 0.118 |

### Gastrointestinal Symptoms (GIS)

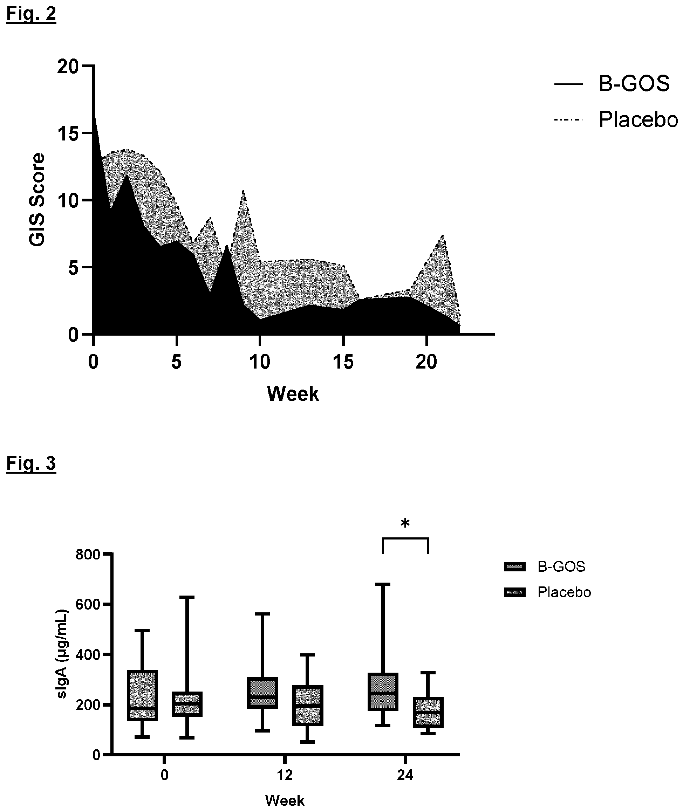

No differences in GIS over the previous 7-days prior to day 0 were evident between the two groups (P = 0.53). A Mann-Whitney U test revealed the AUC of total weekly symptom scores was lower in the B-GOS group (50 [10.5-139.5]) compared to the placebo group (149 [69-208]) (P = 0.03) (Fig. 2). AUC weekly upper symptom scores were lower in the B-GOS group compared to the placebo groups (P < 0.001), but no difference was found for lower GIS (P = 0.11) (data not shown). The number of symptom free weeks for total GIS and upper GIS was lower in the B-GOS group (P < 0.05) compared to placebo group, no difference was evident for lower GIS (P = 0.15) (see Table 7 which shows symptom free weeks for GIS during 24-week study).

**Table 7**

| **Symptoms** | **B-GOS** | | **Placebo** | | **Mean Difference** | **P-values** |
|---|---|---|---|---|---|---|
| | N | Mean (SD) | N | Mean (SD) | 95% Conf. Interval | t-test |
| **Total GI (symptom free weeks)** | 16 | 11 (5.2) | 16 | 7 (4.9) | 3.9 (0.23; 7.52) | 0.04 |
| **Upper GI (symptom free weeks)** | 16 | 15 (2.7) | 16 | 11 (3.6) | 3.8 (1.53; 6.09) | 0.002 |
| **Lower GI (symptom free weeks)** | 16 | 11 (5.0) | 16 | 9 (4.9) | 2.6 (-1.0; 6.2) | 0.15 |

### Systemic inflammation

No differences were observed in CRP or TNF- α at any time points between the two groups (P > 0.05).

### slgA

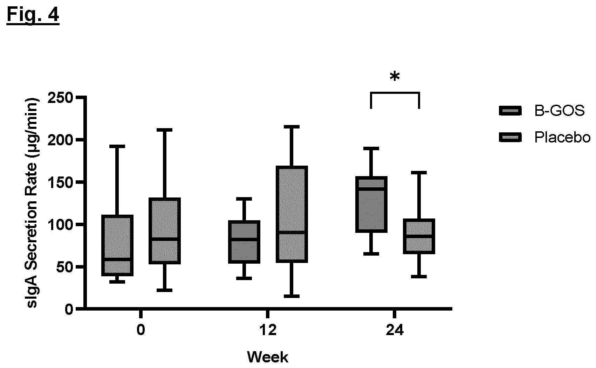

No differences between the two study groups were observed in slgA concentration at week 0 or 12 but at week 24 slgA was greater in the B-GOS group (B-GOS, Median (IQR) 245 (177.9, 319.2) compared to the placebo group, (placebo Median (IQR) 169 (110.6, 229.9); P=0.006) (Fig. 3). No differences were observed in slgA secretion rates at week 0 or 12, but differences were observed at week 24 (B-GOS, Median (IQR) 142 µg/min (93.9, 155.0) vs (Placebo, 86 µg/min (71.5, 105.5); P=0.01) (Fig. 4).

### Conclusions for prebiotic interventions

The main finding of this study was that daily supplementation with B-GOS reduced the duration of URS and the incidence of GI symptoms in elite rugby union players over a 24-week period. Furthermore, B-GOS increased slgA concentrations and secretion rate at 24-weeks when compared to the placebo group. These findings show that B-GOS can modulate the immune and GI system and suppress URS and GI discomfort associated with elite rugby union playing.

Illness can have detrimental effects on athlete training availability and match preparation (Cunniffe et al., 2009; Tiernan et al., 2020; Keaney et al., 2021). To the inventor's knowledge this is the first study to assess the effect of a prebiotic dietary intervention on URS and GIS in an athletic population. The 22% reduction in URS episode duration is clinically significant and relevant for athletes and coaches, ensuring athletes can return to play sooner following a URS episode.

B-GOS has previously been shown to encourage the growth of bifidobacteria in the human gut and subsequently confer numerous health benefits such as reduced systemic inflammation and improved immune response in elderly and overweight populations (Vulevic et al., 2008; Vulevic et al., 2013). Further, increases in faecal short chained fatty acids (SCFA) production, and potential enhancement in epithelial integrity and mucosal immunity have also been reported (Hernot et al., 2009; Mariadason, Barkla & Gibson, 1997). Indeed, in the current study B-GOS increased slgA concentrations and elevated secretion rates at 24-weeks when immunity may have been compromised due to increased training load as seen by a reduction in the placebo group. This suggests that positive manipulation of the gut microbiome may support mucosal immunity and slgA production.

It is believed that B-GOS reduces systemic inflammation through the increased growth of bifidobacteria. Bifidobacteria may amplify dendritic cell sampling in the gut, altering naïve T-cell differentiation and increasing T-cells expressing forkhead box protein P3 (FoxP3), creating an anti-inflammatory effect (McLoughlin & Mills, 2011).

It should be noted that the total number of URS episodes in the present study were lower than those previously reported for the winter season. Elite rugby union players were reported to have experienced four URS episodes per season (Cunniffe et al., 2009), with the greatest incidence during winter. We reported significantly lower rates with greater incidences in the early part of the season. This may explain why B-GOS had little influence on URS incidence and severity. Though, it does suggest B-GOS can still improve URS duration even when incidence rates are low. We also found that participants continued to train despite showing URS. This finding has been found elsewhere in a similar cohort and may be because the participants were required to train when at the training site (Cunniffe et al., 2009). They may also fear the possibility of being deselected from the upcoming match. This is a limitation of collecting self-reported data, future research should determine infections using molecular testing. Another limitation was the frequency of slgA and cytokine measurements. It is possible that B-GOS reduced the duration of URS through enhancement of slgA.

In conclusion, twenty-four weeks of a prebiotic B-GOS supplementation reduced the duration of URS, the incidence and severity of GIS and enhanced slgA secretory rate in elite rugby union players. These findings indicate that B-GOS has the potential to modulate immune function and reduce illness, which improves an athlete's availability to train and compete.

Although a few preferred embodiments have been shown and described, it will be appreciated by those skilled in the art that various changes and modifications might be made without departing from the scope of the invention, as defined in the appended claims.

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the invention. Typically, when referring to compositions, a composition consisting essentially of a set of components will comprise less than 5% by weight, typically less than 3% by weight, more typically less than 1% by weight of non-specified components.

The term "consisting of" or "consists of" means including the components specified but excluding addition of other components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

For the avoidance of doubt, wherein amounts of components in a composition are described in wt%, this means the weight percentage of the specified component in relation to the whole composition referred to. For example, "wherein the oligosaccharide compounds comprise up to 35 wt% of disaccharides" means that 35 wt% of the oligosaccharide compounds in the composition is provided by disaccharides.

The optional features set out herein may be used either individually or in combination with each other where appropriate and particularly in the combinations as set out in the accompanying claims.

## Claims

1. A composition comprising oligosaccharide compounds for use in the prevention, amelioration or treatment of one or more of the following upper Gastro-Intestinal (GI) conditions and/or diseases and Upper Respiratory System (URS) conditions and/or diseases: indigestion, food impaction, swallowing difficulties, heartburn, acid reflux, reflux esophagitis, gastroesophogeal reflux disease, gastritis, small intestine bacterial overgrowth, dyspepsia, bacterial respiratory infections, viral respiratory infections, colds, influenza (flu), respiratory allergies, asthma, headache, chilliness, malaise, coughing, sneezing, sore throat, nasal obstruction, and/or upper respiratory tract infection (URTI), wherein the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},
based on the total weight of oligosaccharide compounds present in the composition;
wherein X^{a}, X^{b} and X^{c} are each independently selected from monosaccharides.

2. The composition for use according to claim 1, wherein the oligosaccharide compounds comprise:
(d) at least 3 wt% Gal-(β1-3)-Gal-(β1-3)-Gal-(β1-4)-X^{d},
based on the total weight of oligosaccharide compounds present in the composition;
wherein X^{d}, is selected from monosaccharides.

3. The composition for use according to claim 1 or claim 2, wherein:
component (a) is present in an amount up to 35 wt%;
component (b) is present in an amount up to 25 wt%; and
component (c) is present in an amount up to 25 wt%;
based on the total weight of oligosaccharide compounds present in the composition.

4. The composition for use according to any one of the preceding claims, wherein the ratio of the wt% of compound (a) to compound (b) is from 1:1 to 3:1 and/or
wherein the ratio of the wt% of compound (a) to compound (c) is from 1:1 to 3:1 and/or
wherein the ratio of the wt% of compound (b) to compound (c) is from 2:1 to 1:2.

5. The composition for use according to any one of the preceding claims, wherein the oligosaccharide compounds comprise:
(e) at least 5 wt% Gal-(β1-4)-Gal-(β1-4)-X^{e};
based on the total weight of oligosaccharide compounds present in the composition;
wherein X^{e} is selected from monosaccharides.

6. The composition for use according to any one of the preceding claims, wherein X^{a}, X^{b}, X^{c}, X^{d} and X^{e} are each independently selected from glucose, fucose, arabinose, xylose, rhamnose, mannose, galactose, ribose, lyxose, allose, altrose, gulose, idose, talose, psicose, fructose, sorbose, tagatose, galactosamine, glucosamine and N-Acetylglucosamine or mixtures thereof.

7. The composition for use according to any one of claims 1 to 5, wherein X^{a}, X^{b}, X^{c}, X^{d} and X^{e} are each Glc or each comprise Fuc.

8. The composition for use according to any one of the preceding claims, wherein from 40 to 55% of the Gal-Gal linkages in the oligosaccharide compounds are 1-3 linkages.

9. The composition for use according to any one of the preceding claims, comprising at least 50 wt% of the oligosaccharide compounds, preferably wherein the composition is in the form of a syrup and/or comprising at least 75 wt% of the oligosaccharide compounds, preferably wherein the composition is in the form of a powder.

10. The composition for use according to any one of the preceding claims, wherein the oligosaccharide compounds comprise at least 25 wt% of trisaccharides and/or wherein the oligosaccharide compounds comprise at least 10 wt% of tetrasaccharides.

11. The composition for use according to any one of the preceding claims, comprising 80 wt% of trisaccharides, tetrasaccharides and higher oligosaccharides and/or comprising 80 wt% of trisaccharides.

12. The composition for use according to any one of the preceding claims, wherein the composition is in the form of a dietary supplement.

13. The composition for use according to any one of claims 1 to 11, wherein the composition is in the form of a pharmaceutical or nutraceutical, optionally wherein the pharmaceutical or nutraceutical further comprises at least one carrier, excipient, or diluent.

14. The composition for use according to any one of the preceding claims, wherein the URS and GI condition and/or disease is in an individual who is an athlete and/or is undergoing, or about to undergo, physical training.

## Patentansprüche

1. Zusammensetzung, die Oligosaccharidverbindungen umfasst, zur Verwendung bei der Prävention, Linderung oder Behandlung einer oder mehrerer der folgenden Leiden und/oder Erkrankungen des oberen Gastrointestinaltrakts (GI) und Leiden und/oder Erkrankungen der oberen Atemwege (URS): Verdauungsstörung, Lebensmitteleinklemmung, Schluckbeschwerden, Sodbrennen, Säurereflux, Refluxösophagitis, gastroesophogeale Refluxerkrankung, Gastritis, bakterielle Überwucherung im Dünndarm, Dyspepsie, bakterielle Atemwegsinfektionen, virale Atemwegsinfektionen, Erkältungen, Influenza (Grippe), Atemwegsallergien, Asthma, Kopfschmerz, Frösteln, Unwohlsein, Husten, Niesen, Halsschmerz, Nasenobstruktion und/oder Infektion der oberen Atemwege (URTI), wobei die Oligosaccharidverbindungen umfassen:
(a) wenigstens 8 Gew.-% Gal-(β1-3)-Gal-(β1-4)-X^{a};
(b) wenigstens 3 Gew.-% Gal-(β1-3)-Gal-(β1-3)-X^{b}; und
(c) wenigstens 5 Gew.-% Gal-(β1-3)-Gal-(β1-2)-X^{c}, bezogen auf das Gesamtgewicht der in der Zusammensetzung vorhandenen Oligosaccharidverbindungen;
wobei X^{a}, X^{b} und X^{c} jeweils unabhängig aus Monosacchariden ausgewählt sind.

2. Zusammensetzung zur Verwendung ach Anspruch 1, wobei die Oligosaccharidverbindungen umfassen:
(d) wenigstens 3 Gew.-% Gal-(β1-3)-Gal-(β1-3)-Gal-(β1-4)-X^{d},
bezogen auf das Gesamtgewicht der in der Zusammensetzung vorhandenen Oligosaccharidverbindungen;
wobei X^{d} aus Monosacchariden ausgewählt ist.

3. Zusammensetzung zur Verwendung nach Anspruch 1 oder Anspruch 2, wobei
Komponente (a) in einer Menge bis zu 35 Gew.-% vorhanden ist;
Komponente (b) in einer Menge bis zu 25 Gew.-% vorhanden ist; und
Komponente (c) in einer Menge bis zu 25 Gew.-% vorhanden ist;
bezogen auf das Gesamtgewicht der in der Zusammensetzung vorhandenen Oligosaccharidverbindungen.

4. Zusammensetzung zur Verwendung nach einem der vorstehenden Ansprüche, wobei das Verhältnis der Gew.-% von Verbindung (a) zu Verbindung (b) von 1:1 bis 3:1 beträgt und/oder
wobei das Verhältnis der Gew.-% von Verbindung (a) zu Verbindung (c) von 1:1 bis 3:1 beträgt und/oder wobei das Verhältnis der Gew.- % von Verbindung (b) zu Verbindung (c) von 2:1 bis 1:2 beträgt.

5. Zusammensetzung zur Verwendung nach einem der vorstehenden Ansprüche, wobei die Oligosaccharidverbindungen umfassen:
(e) wenigstens 5 Gew.-% Gal-(β1-4)-Gal-(β1-4)-X^{e}; bezogen auf das Gesamtgewicht der in der Zusammensetzung vorhandenen Oligosaccharidverbindungen;
wobei X^{e} aus Monosacchariden ausgewählt ist.

6. Zusammensetzung zur Verwendung nach einem der vorstehenden Ansprüche, wobei X^{a}, X^{b}, X^{c}, X^{d} und X^{e} jeweils unabhängig ausgewählt sind aus Glucose, Fucose, Arabinose, Xylose, Rhamnose, Mannose, Galactose, Ribose, Lyxose, Allose, Altrose, Gulose, Idose, Talose, Psicose, Fructose, Sorbose, Tagatose, Galactosamin, Glucosamin und N-Acetylglucosamin und Gemischen davon.

7. Zusammensetzung zur Verwendung nach einem der Ansprüche 1 bis 5, wobei Xa^{,} X^{b}, X^{c}, X^{d} und X^{e} jeweils Glc sind oder jeweils Fuc umfassen.

8. Zusammensetzung zur Verwendung nach einem der vorstehenden Ansprüche, wobei 40 bis 55 % der Gal-Gal-Verknüpfungen in den Oligosaccharidverbindungen 1-3-Verknüpfungen sind.

9. Zusammensetzung zur Verwendung nach einem der vorstehenden Ansprüche, umfassend wenigstens 50 Gew.-% an den Oligosaccharidverbindungen, wobei die Zusammensetzung vorzugsweise in Form eines Sirups vorliegt und/oder wenigstens 75 Gew.-% an den Oligosaccharidverbindungen umfasst, wobei die Zusammensetzung vorzugsweise in Form eines Pulvers vorliegt.

10. Zusammensetzung zur Verwendung nach einem der vorstehenden Ansprüche, wobei die Oligosaccharidverbindungen wenigstens 25 Gew.-% Trisaccharide umfassen und/oder wobei die Oligosaccharidverbindungen wenigstens 10 Gew.-% Tetrasaccharide umfassen.

11. Zusammensetzung zur Verwendung nach einem der vorstehenden Ansprüche, umfassend 80 Gew.-% Trisaccharide, Tetrasaccharide und höhere Oligosaccharide und/oder umfassend 80 Gew.-% Trisaccharide.

12. Zusammensetzung zur Verwendung nach einem der vorstehenden Ansprüche, wobei die Zusammensetzung in Form eines Nahrungsergänzungsmittels vorliegt.

13. Zusammensetzung zur Verwendung nach einem der Ansprüche 1 bis 11, wobei die Zusammensetzung in Form eines Arzneimittels oder Nutrazeutikums vorliegt, gegebenenfalls wobei das Arzneimittel oder Nutrazeutikum ferner wenigstens einen Träger, Exzipienten oder Verdünnungsmittel umfasst.

14. Zusammensetzung zur Verwendung nach einem der vorstehenden Ansprüche, wobei das/die URS- und GI-Leiden und/oder Erkrankung in einem Individuum vorliegt, das ein Athlet ist und/oder sich einem körperlichen Training unterzieht oder bald unterzieht.

## Revendications

1. Composition comprenant des composés oligosaccharides pour une utilisation dans la prévention, l'amélioration ou le traitement d'une ou plusieurs des affections et/ou maladies gastro-intestinales (GI) supérieures et des affections et/ou maladies du système respiratoire supérieur (URS) suivantes : indigestion, impaction alimentaire, difficultés à avaler, brûlures d'estomac, reflux acide, œsophagite par reflux, reflux gastro-œsophagien, gastrite, prolifération bactérienne de l'intestin grêle, dyspepsie, infections respiratoires bactériennes, infections respiratoires virales, rhume, grippe, allergies respiratoires, asthme, maux de tête, frissons, malaise, toux, éternuement, maux de gorge, obstruction nasale et/ou infection des voies respiratoires supérieures (URTI), dans laquelle les composés oligosaccharides comprennent :
(a) au moins 8 % en poids de Gal-(β1-3)-Gal-(β1-4)-X^{a} ;
(b) au moins 3 % en poids de Gal-(β1-3)-Gal-(β1-3)-X^{b} ; et
(c) au moins 5 % en poids de Gal-(β1-3)-Gal-(β1-2)-X^{c}, par rapport au poids total de composés oligosaccharides présents dans la composition ;
X^{a}, X^{b} et X^{c} étant chacun indépendamment choisis parmi les monosaccharides.

2. Composition pour une utilisation selon la revendication 1, dans laquelle les composés oligosaccharides comprennent :
(d) au moins 3 % en poids de Gal-(β1-3)-Gal-(β1-3)-Gal-(β1-4)-X^{d},
par rapport au poids total de composés oligosaccharides présents dans la composition ;
X^{d}, étant choisi parmi des monosaccharides.

3. Composition pour une utilisation selon la revendication 1 ou la revendication 2, dans laquelle :
le composant (a) est présent en une quantité allant jusqu'à 35 % en poids ;
le composant (b) est présent en une quantité allant jusqu'à 25 % en poids ; et
le composant (c) est présent en une quantité allant jusqu'à 25 % en poids ;
par rapport au poids total de composés oligosaccharides présents dans la composition.

4. Composition pour une utilisation selon l'une quelconque des revendications précédentes, dans laquelle le rapport du % en poids du composé (a) à celui du composé (b) va de 1:1 à 3:1 et/ou
dans laquelle le rapport du % en poids du composé (a) à celui du composé (c) va de 1:1 à 3:1 et/ou dans laquelle le rapport du % en poids du composé (b) à celui du composé (c) va de 2:1 à 1:2.

5. Composition pour une utilisation selon l'une quelconque des revendications précédentes, dans laquelle les composés oligosaccharides comprennent :
(e) au moins 5 % en poids de Gal-(β1-4)-Gal-(β1-4)-X^{e} ; par rapport au poids total de composés oligosaccharides présents dans la composition ;
X^{e} étant choisi parmi les monosaccharides.

6. Composition pour une utilisation selon l'une quelconque des revendications précédentes, dans laquelle X^{a}, X^{b}, X^{c}, X^{d} et X^{e} sont chacun indépendamment choisis parmi le glucose, le fucose, l'arabinose, le xylose, le rhamnose, le mannose, le galactose, le ribose, le lyxose, l'allose, l'altrose, le gulose, l'idose, le talose, le psicose, le fructose, le sorbose, le tagatose, la galactosamine, la glucosamine, la N-acétylglucosamine ou les mélanges de ceux-ci.

7. Composition pour une utilisation selon l'une quelconque des revendications 1 à 5, dans laquelle X^{a}, X^{b}, X^{c}, X^{d} et X^{e} sont chacun Glc ou comprennent chacun Fuc.

8. Composition pour une utilisation selon l'une quelconque des revendications précédentes, dans laquelle de 40 à 55 % des liaisons Gal-Gal dans les composés oligosaccharides sont des liaisons 1-3.

9. Composition pour une utilisation selon l'une quelconque des revendications précédentes, comprenant au moins 50 % en poids des composés oligosaccharides, de préférence dans laquelle la composition est sous la forme d'un sirop et/ou comprenant au moins 75 % en poids des composés oligosaccharides, de préférence dans laquelle la composition est sous la forme d'une poudre.

10. Composition pour une utilisation selon l'une quelconque des revendications précédentes, dans laquelle les composés oligosaccharides comprennent au moins 25 % en poids de trisaccharides et/ou dans laquelle les composés oligosaccharides comprennent au moins 10 % en poids de tétrasaccharides.

11. Composition pour une utilisation selon l'une quelconque des revendications précédentes, comprenant 80 % en poids de trisaccharides, tétrasaccharides et oligosaccharides supérieurs et/ou comprenant 80 % en poids de trisaccharides.

12. Composition pour une utilisation selon l'une quelconque des revendications précédentes, dans laquelle la composition est sous la forme d'un complément alimentaire.

13. Composition pour une utilisation selon l'une quelconque des revendications 1 à 11, dans laquelle la composition est sous la forme d'un produit pharmaceutique ou nutraceutique, éventuellement dans laquelle le produit pharmaceutique ou nutraceutique comprend en outre au moins un véhicule, excipient ou diluant.

14. Composition pour une utilisation selon l'une quelconque des revendications précédentes, dans laquelle l'affection et/ou maladie URS et GI est chez un individu qui est un athlète et/ou qui subit, ou est sur le point de subir, un entraînement physique.
